# EUROPEAN PATENT APPLICATION

(11) **EP 1 129 702 A1**
(43) Date of publication of application: **05.09.2001**
(21) Application number: 00200662.5
(22) Date of filing: 25.02.2000
(51) Int. Cl.: A61K 7/50

(54) **Shower gel**

(71) Applicant: Sara Lee/DE N.V., 3532 AA Utrecht (NL)
(72) Inventor: Withell, Trevor Keith, 40489 Düsseldorf (DE)
(74) Representative: Ottevangers, Sietse Ulbe

(57) **Abstract**

The present invention is directed to a pressurised container, containing a shower gel composition, said composition comprising suitable shower gel components and, based on the weight of the composition, at least 40 % of a low boiling, pressurised gas.

## Description

The present invention relates to cosmetic compositions in the form of a shower gel in a pressurised container.

Consumers differentiate between cosmetic compositions by evaluating many different elements of the product. These factors include marketing, advertising, claimed effects and in-use performance. A key element of the in-use performance is the way a product composition feels on the skin or hair whilst the product is being applied and used. Developing new and unique sensations for products is a major objective of most new product development programmes to ensure different performance from other products.

Cosmetic compositions which are designed to cleanse the skin or hear, use the properties of the foam generated by using surfactants to convey the type of cleansing, conditions and other properties of the composition. This effect of foaming is generally considered important in the appreciation of the product by the consumers.

Cosmetic composition which are designed to refresh and cool the skin or hair use a variety of different materials to achieve this effect. One such system contains mixture of butane, water, ethanol and other minor ingredients to give a cooling and fizzing effect on the skin or hair. In these systems butane is usually present in amounts normally greater than 50% wt. This system relies upon the rapid boiling of the butane upon contact with the skin, the consequence of which is a highly perceived sensation on the skin.

In these two examples of properties of cosmetic compositions, the physical chemistry of the effects (foaming, fizzing) are generally regarded as being mutually exclusive. The former effect of foaming relies upon the use of a liquid that has a stable interfacial film to generate the bubbles. The latter effect of fizzing requires a liquid that cannot form stable interfacial films, enabling bubble of entrapped butane to escape rapidly from the liquid and burst at the surface.

The present invention seeks to provide a composition which has both the properties of a foaming cleansing product with a rich and appreciated foam whilst on application to the skin is refreshing and has a strongly perceived fizzing effect. Surprisingly, the present invention has solved the previously accepted mutual exclusivity of foaming and fizzing effects on the skin by separating these events in time.

In accordance with the present invention there is provided a pressure container, containing a shower gel composition, said composition comprising suitable shower gel components and, based on the weight of the composition, at least 40 % of a low boiling, pressurised gas.

The present invention is based on the surprising appreciation that by the use of a substantial amount of pressurised gas in a conventional shower gel composition, the combined effect of fizzing and foaming can be obtained.

When using the shower gel composition of the present invention, the gel from the container will be applied by opening the valve, resulting in an amount of gel being emitted from the container. When this gel comes into contact with the skin, the pressurised gas rapidly boils giving the fizzing and cooling sensation. Subsequently, when the gels comes into contact with water, the foaming effect occurs.

A composition according to the invention accordingly contains on the one hand the conventional shower gel components and on the other hand a pressurised gas or rapidly boiling gas, which boils upon contact with the skin. Preferably, the composition contains a combination of surfactants, alcohol, a thickener, other required materials, such as colour and perfume, and water contained in a aerosol package. It is preferred that the present composition has the form of an emulsion, having the viscosity of a body lotion, which on application to the skin fizzes until the majority of the pressurised gas has boiled. The resulting composition is subsequently mixed with the water used for washing to produce a rich, creamy lather. Suitable pressurised gases include propane, isopropane, butane, isobutane, pentane, isopentane and dimethyl ether present in an amount from 40% to 80% based on the weight of the composition. Optimised compositions more typically contain 50% to 70% of the pressurised gas.

A further important consideration of the present invention is the concentration of water in the composition. Preferably, the water content is less than 20%, ideally in a range from 2 to 15%. Optimised formulations according to the present invention contain 10 to 13% water to ensure that foaming is not activated until additional water is added in the washing process, but enough water is included to make the composition as cost effective as possible.

The composition further comprises one or more surfactants. Preferably, the surfactant or surfactants are foaming and skin friendly. Examples of suitable surfactants include polysorbate 20 or 40, coc-glucoside, lauryl glucoside, decyl glucoside, lauryl sulphates, lauryl ether sulphates such as ammonium, sodium, magnesium, MEA, TEA, MIPA or TIPA laureth sulphate, cocamidopropylbetain or sodium alkyl sulphosuccinates. The surfactants are preferably present in amounts from 2 to 15% wt., preferably from 5 to 10 % wt., based on the weight of the composition.

Another preferred component of the composition is alcohol, examples of which include ethanol, isopropanol, n-propanol, n-butanol, isobutanol and t-butanol. The alcohol serves to moderate the fizzing effect either by slowing the rate of boiling of the pressurised gas or by changing the perceived cooling effect on the skin or hair. The alcohol is preferably present in amounts from 8% to 20% based on the weight of the composition. Optimised compositions contain between 10% and 15% of an alcohol based on the weight of the composition.

The final critical component of the composition is a thickener selected to provide stability of the emulsion as applied to the skin but must also allow the interfacial film to rupture whilst the pressurised gas is boiling. Examples of such thickeners include cross-linked polyacrylates, acrylates and copolymers thereof, cellulose derivatives, such as hydroxy ethylcellulose, hydroxymethylcellulose, hydroxyethyl-methylcellulose, hydroxypropylcellulose, polyvinylpyrrolidone and copolymers thereof, xanthan gum, synthetic hectorite (Laponite™), synthetic silicates (Optigel™) and inorganic salts such as sodium chloride and ammonium sulphate. Preferred examples contain 0.1% to 5% of the thickener based on the weight of the composition. Optimised formulations contain 1% to 2% of a synthetically produced magnesium silicate.

Depending on the envisaged purpose of the composition, one or more other ingredients may be present. Examples of such ingredients include preservatives, anti-bacterial agents, pH regulating agents, emollients, perfumes, moisturising agents, colourants and UV filters. The composition is formulated to be an emulsion that enables the pressurised gas to evaporate rapidly to give a fizzing effect and subsequently performs as an acceptable washing product leaving the skin or hair in an acceptable state as regards the way it feels.

The pH of the composition is preferably regulated to be close to the pH of skin itself. Accordingly, the pH of the composition is preferably slightly acidic, e.g. in the range of 5 to 8. An example of a suitable pH regulating agent is citric acid. The skilled person will be aware of numerous suitable pH regulating agents that may be employed in the present type of compositions. The amount of the pH regulating agent present is of course adjusted so that the desired pH is achieved.

An emollient or moisturising agent may be present in order to improve the ease of application to the skin of the composition and the final skin feel the user experiences. Examples of suitable emollients or moisturising agents include glycerin, propylene glycol, PEG-7 glyceryl cocoate, PEG-6 caprylic or capric glycerides, glyceryl oleate and lipids in general, such as paraffin oil or polar oils. An emollient or moisturising agent is preferably present in an amount ranging from 0.5 to 15% wt., based on the weight of the composition.

The balance of the composition will generally be made up by the pressurised gas.

The shower gel of the present invention can be prepared in the conventional manner for preparing comparable shower gel compositions. According to a preferred embodiment first water and a thickener glycerol mixture are prepared by adding the thickener composition to the water. Subsequently, the various components are added one by one and mixed into the material. Finally, the pH is adjusted to the required value and the final product is filled into the container, optionally some alcohol is added after which the can is closed. At that stage the pressurised gas is added to the can and the product is ready.

The present invention is now elucidated by the following, non-restricted example.

### Example

A composition was prepared of the following ingredients in the following amounts (%wt.);
- Surfactants: TIPA Laureth Sulphate 5.3
   Polysorbate 20 2.5
- Water Water 11.5
- Thickener Magnesium Silicate 1.5
- Moisturising Agent Glycerin 7.5
- Alcohol Denatured Ethanol 10.5
- Pressurised Gas Butane to 100 %wt

## Claims

1. Pressurised container, containing a shower gel composition, said composition comprising suitable shower gel components and, based on the weight of the composition, at least 40 % of a low boiling, pressurised gas.

2. Container according to claim 1, wherein the water content of the gel is at most 20 wt.%.

3. Container according to claim 1 or 2, wherein the water content is between 2 and 15 wt.%.

4. Container according to claims 1-3, wherein the amount of pressurised gas is between 50 and 80 wt.%.

5. Container according to claims 1-4, wherein the said pressurised gas is selected from the group of propane, isopropane, butane, iso-butane, pentane, iso-pentane, dimethylether and mixtures of two or more of these, preferably butane.

6. Container according to claims 1-5, wherein further at least one alcohol is present, preferably in a amount of from 8 to 20 wt.% of the composition.

7. Container according to claim 6, wherein the said alcohol is selected from the group of ethanol, isopropanol, n-propanol, n-butanol, t-butanol, iso-butanol, and mixtures of two or more of these.

8. Container according to claims 1-7, wherein the pH of the shower gel composition is between 5 and 8.

9. Container according to any one of the preceding claims, wherein the gel composition further comprises at least one component selected from the group of surfactants, thickeners, preservatives, anti-bacterial agents, pH-regulating agents, emollients, perfumes, moisturising agents, colorants and UV filters.

10. Container according to claims 1-9, wherein the said composition comprises in wt.%:
40-80 % of pressurised gas.
